# EUROPEAN PATENT SPECIFICATION

(11) **EP 4 531 765 B1**
(45) Date of publication and mention of the grant of the patent: **01.07.2026**
(21) Application number: 23731341.6
(22) Date of filing: 23.05.2023
(51) Int. Cl.: A61F 2/46, A61F 2/44, A61B 17/02

(54) **INSERTION INSTRUMENTS**
EINSETZINSTRUMENTE
INSTRUMENTS D'INSERTION

(30) Priority: 25.05.2022 GB 202207714
(43) Date of publication of application: 09.04.2025
(73) Proprietor: Axis Spine Technologies Ltd, Leeds LS15 8ZB (GB)
(72) Inventor: ARCOS, Jonathan, Apsley Hemel Hempstead Hertfordshire HP3 9SQ (GB); HEUER, Frank, Apsley Hemel Hempstead Hertfordshire HP3 9SQ (GB)
(74) Representative: Peter, Kenneth William
(86) International application number: PCT/GB2023/051350
(87) International publication number: WO 2023/227877

(56) References cited:
- WO-A1-2014/145766
- WO-A1-2021/014173
- WO-A1-2021/014176
- WO-A1-2021/058971

## Description

### Field of the Invention

The present invention relates to insertion instruments for inserting an Oblique Lumbar Interbody Fusion (OLIF) device into an intervertebral space, and more particularly but not exclusively for inserting a modular OLIF device into an intervertebral space.

### Background Art

Adjacent vertebrae in the spinal column are coupled to each other by a number of ligaments and the intervertebral disc. These anatomic structures hold the adjacent vertebrae together while allowing motion. Among these structures, the intervertebral disc functions as a cushion between the vertebrae whilst allowing for relative movement of the vertebrae. Problems with intervertebral discs arise from one or more of a range of diseases and conditions. A surgical procedure, such as spinal fusion, may be used to address such problems. The goals of spinal fusion include decompressing surrounding neural structures, re-establishing anatomic spinal alignment and stabilising the motion segment by having one vertebral body fuse, or heal, to the adjacent vertebral body. A typical spinal fusion procedure involves partial or full removal of a problematic intervertebral disc and installation of an intervertebral device in the place of the partially or fully removed intervertebral disc in order to maintain the intervertebral space height and alignment and facilitate the fusion of one vertebra to the next.

A known form of intervertebral device is the modular intervertebral device which comprises superior and inferior plates and a core component. The superior and inferior plates and a core component are separate components. The core component is sized and shaped to determine a separation between the superior and inferior plates. When the superior and inferior plates face each other, the core component is inserted between the superior and inferior plates to bring adjacent ones of the superior and inferior plates and the core component into engagement.

Considering spinal fusion procedures further, at least one incision is made in the patient's body and a path to the spine is cleared by retraction of intervening tissue. Then the problematic disc is removed from the intervertebral space and the intervertebral space is prepared properly before the intervertebral device is introduced into the prepared intervertebral space. Introduction of the intervertebral device into the intervertebral space is often accomplished by way of an insertion instrument. Usually, the intervertebral device is held by the insertion instrument with the surgeon using the insertion instrument to introduce the intervertebral device into the abdomen, guide the intervertebral device through the abdomen, and then insert the intervertebral device into the intervertebral space.

A known form of insertion instrument has two arms which extend from the end of the insertion instrument held by the surgeon with the distal end of each arm engaging a respective one of the superior and inferior plates of a modular intervertebral device. The arms of the insertion instrument are movable relative to each other. When the superior and inferior plates have been introduced into the intervertebral space, the distal ends of the arms are moved by the surgeon to achieve a desired height and/or angle for the intervertebral device. The core component is then inserted between the superior and inferior plates. Insertion of the core component is by way of a so-called core loader which may be comprised in the insertion instrument.

A spinal fusion procedure may be carried out by way of one of several different techniques. Posterior Lumbar Interbody Fusion (PLIF) is one such technique in which the patient's spine is approached from an incision in the middle of the back. Anterior Lumbar Interbody Fusion (ALIF) is another such technique in which the patient's spine is approached from the opposite direction, i.e. from an incision on the anterior side of the patient. An example thereof is disclosed in WO 2021/014173 A1.

A third such technique is Oblique Lumbar Interbody Fusion (OLIF) in which the patient's spine is approached from an incision between the middle of the stomach and the side of the body to thereby provide an oblique trajectory for insertion of the intervertebral device. Normally, the incision is made such that a path between the incision and the part of the spine to be treated is at an angle of between 30 degrees and 60 degrees to the coronal or sagittal plane. OLIF minimises cutting of muscles and involves use of a single port to access the intervertebral space whereby OLIF can be performed as a minimally invasive fashion.

Investigations by or on behalf of the present inventors have shown known insertion instruments to present a problem for insertion of modular OLIF intervertebral devices. A wedge shaped core component slopes in the coronal or sagittal plane. If a known insertion instrument is used to insert a PLIF or ALIF intervertebral device which has a wedge shaped core component, the arms of the insertion instrument are able to orientate the superior and inferior plates appropriately relative to each other for reception of the wedge shaped core component. However, when insertion of an OLIF intervertebral device is attempted during an OLIF procedure, the known inserter is unable to orientate the superior and inferior plates appropriately relative to each other for proper reception of the wedge shaped core component between the superior and inferior plates. This is because the arms of the insertion instrument extend in a direction oblique to the coronal and sagittal planes and therefore provide for relative orientation of the superior and inferior plates with reference to the oblique direction whereas the wedge shaped core component slopes in the coronal or sagittal plane.

The present invention has been devised in light of the above problem. It is therefore an object for the present invention to provide an insertion instrument for inserting an Oblique Lumbar Interbody Fusion (OLIF) device into an intervertebral space.

### Statement of Invention

The present invention relates to an insertion instrument and kit as claimed hereafter. Preferred embodiments of the invention are set forth in the dependent claims. While the implantation methods described herein do not form part of the invention, they are disclosed as they represent useful background for understanding the invention.

According to a first aspect of the present invention there is provided an insertion instrument for inserting a modular Oblique Lumbar Interbody Fusion (OLIF) device into an intervertebral space, the insertion instrument comprising:
a hand grippable support defining a longitudinal axis;
a first elongate arm mounted at a proximal end thereof on the hand grippable support and extending from the hand grippable support along the longitudinal axis, the first elongate arm configured at a distal end thereof to grip a superior plate of the modular OLIF device;
a second elongate arm mounted at a proximal end thereof on the hand grippable support and extending from the hand grippable support along the longitudinal axis, the second elongate arm configured at a distal end thereof to grip an inferior plate of the modular OLIF device, the distal ends of the first and second elongate arms opposing each other whereby, in use, an inferior surface of the superior plate and a superior surface of the inferior plate face each other, wherein
the proximal ends of the first and second arms are mounted on the hand grippable support for relative rotation of the first and second arms about the longitudinal axis,
the proximal end of the first arm may have
first and second sides facing away from each other and spaced apart in a direction orthogonal to the longitudinal axis and to a direction of separation of the first and second arms,
the proximal end of the second arm may have
third and fourth sides facing away from each other and spaced apart in a direction orthogonal to the longitudinal axis and to a direction of separation of the first and second arms, the first and third sides on a first side of the insertion instrument, the second and fourth sides on a second side of the insertion instrument, and the proximal ends of the first and second arms may be mounted on the hand grippable support to substantially prevent increase in separation between the first and third sides simultaneously with increase in separation between the second and fourth sides.

The insertion instrument is for inserting a modular Oblique Lumbar Interbody Fusion (OLIF) device into an intervertebral space. The insertion instrument comprises a hand grippable support, and first and second elongate arms mounted on the hand grippable support. In use, a surgeon may hold the insertion instrument by the hand grippable support. The hand grippable support defines a longitudinal axis. The first elongate arm is mounted at its proximal end on the hand grippable support such that the arm extends from the hand grippable support along the longitudinal axis. The first elongate arm is configured at its distal end to grip a superior plate of the modular OLIF device. The second elongate arm is mounted at its proximal end on the hand grippable support such that the arm extends from the hand grippable support along the longitudinal axis. The second elongate arm is configured at its distal end to grip an inferior plate of the modular OLIF device. The distal ends of the first and second elongate arms oppose each other whereby, in use, an inferior surface of the superior plate and a superior surface of the inferior plate oppose each other, such as when the superior and inferior plates are held by the insertion instrument in the intervertebral space.

The proximal ends of the first and second arms are mounted on the hand grippable support for relative rotation of the first and second arms about the longitudinal axis. Relative rotation of the first and second arms about the longitudinal axis may be caused by relative rotation of the distal ends of the first and second arms. Relative rotation of superior and inferior plates gripped by the distal ends of the first and second arms upon insertion of a wedge shaped core component of the OLIF device between the plates may orientate the superior and inferior plates appropriately relative to each other during an OLIF procedure to allow for proper insertion of the core component between the superior and inferior plates.

The proximal end of the first arm may have first and second sides facing away from each other and spaced apart in a direction orthogonal to the longitudinal axis and to a direction of separation of the first and second arms. Furthermore, the proximal end of the second arm may have third and fourth sides facing away from each other and spaced apart in a direction orthogonal to the longitudinal axis and to a direction of separation of the first and second arms. The first and third sides may be on a first side of the insertion instrument, and the second and fourth sides are on a second side of the insertion instrument. The proximal ends of the first and second arms may be mounted on the hand grippable support to substantially prevent increase in separation between the first and third sides simultaneously with increase in separation between the second and fourth sides. Furthermore, the proximal ends of the first and second arms may be mounted on the hand grippable support to substantially prevent decrease in separation between the first and third sides simultaneously with decrease in separation between the second and fourth sides. Thus, when there is increase in separation between the first and third sides upon relative rotation of the first and second arms about the longitudinal axis in a first direction, this is accompanied by decrease in separation between the second and fourth sides. Also, when there is increase in separation between the second and fourth sides upon relative rotation of the first and second arms about the longitudinal axis in a second direction opposite the first direction, this is accompanied by decrease in separation between the first and third sides.

At least one of the first and second arms may be configured at a location along its length for rotation of the distal end of the arm about an arm rotation axis which is orthogonal to the longitudinal axis and to a direction of separation of the first and second arms. Rotation about the arm rotation axis may change separation between the distal ends of the first and second arms. In use, rotation about the arm rotation axis may change a separation of the superior and inferior plates gripped by the insertion instrument. Rotation in a first direction about the arm rotation axis may increase the separation and rotation in a second direction opposite the first direction may decrease the separation.

The first and second arms may be biased to reduce a separation between the distal ends of the first and second arms. In use, and when superior and inferior plates are gripped by the distal ends, the bias may urge the superior and inferior plates together to thereby reduce the profile of the superior and inferior plates. The reduced profile may provide for ease of insertion of the superior and inferior plates into the intervertebral space. When the core component of the OLIF device is then inserted between the superior and inferior plates, as described further below, the superior and inferior plates may be pushed apart against the bias. Bias to reduce separation between the distal ends of the first and second arms may be by way of a spring bias, such as by a spring configured to exert spring bias against one of the first and second arms. Each of the first and second arms may be biased in this fashion.

Each of the first and second arms may be configured at a location along its length for rotation of its distal end about a respective arm rotation axis. Change in separation between the distal ends of the first and second arms may be achieved by one, other or both of rotation about the arm rotation axis of the first arm and rotation about the arm rotation axis of the second arm.

The arm may be configured at or adjacent the proximal end of the arm for rotation about the arm rotation axis. The whole arm may therefore rotate about the arm rotation axis. The insertion instrument may comprise a rotatable coupling which rotates about the arm rotation axis. The rotatable coupling may be a hinge. A first part of the rotatable coupling may be defined by the proximal end of the arm and a second part of the rotatable coupling may be defined by the hand grippable support, the first and second parts coupled for their relative rotation. Where both arms are rotatable, the first arm may comprise a first rotatable coupling and the second arm may comprise a second rotatable coupling. The first and second sides of the first arm may be at opposite ends of the first rotatable coupling and the third and fourth sides of the second arm may be at opposite ends of the second rotatable coupling.

According to the invention, hand grippable support comprises a first support portion on which the first arm is mounted, and a second support portion on which the second arm is mounted, the first and second support portions coupled for their relative rotation about the longitudinal axis. Relative rotation of the first and second support portions therefore causes relative rotation of the first and second arms about the longitudinal axis and vice-versa.

The first and second support portions are coaxially disposed. Furthermore, the second support portion may be contained along a part of its length within a space defined by the first support portion, the contained part of the second support portion rotatable within the space defined by the first support portion. The first support portion may comprise a sleeve which defines a substantially cylindrical space. The contained part of the second support portion may define an external surface of substantially the same circular cross-section along its length, the contained part of the second support portion received snugly within the substantially cylindrical space whereby the contained part is rotatable in the sleeve.

An exterior surface of the sleeve may define a hand grippable surface. The hand grippable surface may be contoured along its length, i.e. along the longitudinal axis, to thereby provide for improved grip by the surgeon.

The first support portion may further comprise a first arm mounting bracket, the first arm mounting bracket attached to and extending from an end of the sleeve. The first arm mounting bracket may be immovably attached to the sleeve. The first arm may therefore rotate about the longitudinal axis when the first support portion is rotated about the longitudinal axis and vice-versa. Furthermore, the first arm may be attached to the first arm mounting bracket, such as by way of the first rotatable coupling described above.

The second support portion may further comprise a second arm mounting bracket, the second arm mounting bracket attached to and extending from an end of the contained part of the second support portion. The second arm mounting bracket may be immovably attached to the contained part of the second support portion. The second arm may therefore rotate about the longitudinal axis when the second support portion is rotated about the longitudinal axis and vice-versa. Furthermore, the second arm may be attached to the second arm mounting bracket, such as by way of the second rotatable coupling described above.

The second support portion may comprise a tube which is received in the sleeve of the first support portion. The tube may thus constitute the contained part of the second support portion whereby the second arm mounting bracket is attached to and extends from an end of the tube. As described below, an inside of the tube may slidably receive a part of a core loader.

The insertion instrument may further comprise an elongate core loader which is configured at its distal end to move a core component of the OLIF device along the longitudinal axis and more specifically is configured at its distal end to support the core component, such as by gripping the core component. In use, the core loader may be used after insertion of the superior and inferior plates into the intervertebral space to insert the core component between the superior and inferior plates.

The core loader may be mounted on the insertion instrument for movement of the core loader relative to the first and second arms along the longitudinal axis whereby a core component gripped by the distal end of the core loader may be pushed into the abdomen and then into the intervertebral space between the superior and inferior plates.

The core loader may be mounted on the insertion instrument such that the distal end of the core loader is between the first and second arms. Furthermore, the core loader may comprise a core loader proximal portion which extends from a proximal end of the core loader along the core loader. The core loader proximal portion may be shaped and sized to be received in and move along the tube of the second support portion. The core loader may thus be moved along the longitudinal axis for insertion of the core component between the superior and inferior plates. In addition, a core loader distal portion which extends from an end of the core loader proximal portion to the distal end of the core loader may be received between the first and second arms.

The core loader proximal portion may define a threaded portion which threadedly engages with the second support portion. Rotation of the core loader proximal portion relative to the second support portion may thus move the core loader along the longitudinal axis. Rotation of the core loader proximal portion may result in an undesirable corresponding rotation of the distal end of the core loader. The core loader may therefore comprise a rotatable coupling between the core loader proximal portion and the core loader distal portion. Furthermore, the core loader distal portion may be configured to resist rotation of the core loader distal portion when the core loader proximal portion is rotated. The core loader distal portion may comprise a rotation resisting formation, such as at least one protrusion, which bears against at least one of the first and second arms. The rotation resisting formation may bear against at least one arm to thereby present resistance to rotation of the core loader distal portion with rotation of the core loader proximal portion. Furthermore, the arm and the rotation resisting formation may be configured such that the rotation resisting formation bears against the arm as the rotation resisting formation moves relative to the arm as the core loader is moved along the longitudinal axis. For example, and where the rotation resisting formation is a protrusion, the arm may define a slot in which the protrusion moves as the core loader moves along the longitudinal axis.

A distal end of an arm comprise two legs which extend along the longitudinal axis, the two legs spaced apart in a direction orthogonal to the longitudinal axis. Each of the two legs may be configured to interengage with a respective one of two recesses on a plate. The distal end of the arm may thus be configured to grip the plate. Each leg may comprise a leg protrusion which extends in a direction orthogonal to the longitudinal axis. The leg protrusion may be near or at a distal end of the leg. The leg protrusions of the two legs may extend towards each other. Furthermore, the leg protrusions may be in registration with each other. Each leg protrusion may, in use, be received in a respective one of two recesses in a plate, the two recesses defined in opposite sides of the plate. The leg protrusions may be shaped for rotation in their respective recesses whereby the plate is rotatable relative to the legs.

At least one of the two legs may be mounted on the distal end of the arm for change in separation between the two legs against a bias and more specifically against a spring bias. The legs may thus engage with and disengage from a plate. The spring bias may be exerted by a leg. The arm may comprise a leg separation member which is mounted for movement thereon and which when moved bears against at least one of the legs to change the separation. Movement of the leg separation member may be along the longitudinal axis and towards the distal ends of the legs. The leg separation member may be elongate and may extend along the arm to a proximal end. The leg separation member may be shaped at its proximal end for engagement with a user's digit whereby the user may press against the shaped proximal end to operate the leg separation member.

Upper and lower sides of a core component of the OLIF device may be inclined to each other. The core component may therefore have the form of a wedge. The insertion instrument may be used to advantage with a wedge shaped core component. Furthermore, the upper side and a lower side may not meet at an acute angle whereby the core component has the form of a frustum of a wedge. The core component and the inferior and superior plates may be configured for insertion of the thinner edge of the thinner and thicker edges of the core component. An inclination of the inferior and superior plates relative to each other may thus be determined by way of the core component further to a separation between the inferior and superior plates. Extent of inclination of the inferior and superior plates may be determined by selection from a plurality of core components having upper and lower sides of different inclinations. Such selection may be combined with selection from a plurality of core components having different heights.

According to a second aspect of the present invention, there is provided a kit for an OLIF procedure comprising an insertion instrument according to the first aspect of the present invention, a superior plate, an inferior plate, and at least one core component. An OLIF device may be constituted by the superior plate, the inferior plate, and the core component. The OLIF device may further comprise at least one screw to secure the OLIF device to anatomy. Alternatively or in addition, the OLIF device may further comprise a cover plate.

Further embodiments of the second aspect may comprise one or more features of the first aspect of the present invention.

According to a further aspect of the present invention, there is provided an insertion instrument for inserting a modular Oblique Lumbar Interbody Fusion (OLIF) device into an intervertebral space, the insertion instrument comprising: a hand grippable support defining a longitudinal axis; a first elongate arm mounted at a proximal end thereof on the hand grippable support and extending from the hand grippable support along the longitudinal axis, the first elongate arm configured at a distal end thereof to grip a superior plate of the modular OLIF device; a second elongate arm mounted at a proximal end thereof on the hand grippable support and extending from the hand grippable support along the longitudinal axis, the second elongate arm configured at a distal end thereof to grip an inferior plate of the modular OLIF device, the distal ends of the first and second elongate arms opposing each other whereby, in use, an inferior surface of the superior plate and a superior surface of the inferior plate oppose each other, wherein the proximal ends of the first and second arms are mounted on the hand grippable support for relative rotation of the first and second arms about the longitudinal axis.

The hand grippable support comprises a first support portion on which the first arm is mounted, and a second support portion on which the second arm is mounted, the first and second support portions coupled for their relative rotation about the longitudinal axis. Relative rotation of the first and second support portions therefore causes relative rotation of the first and second arms about the longitudinal axis. The first and second support portions are coaxially disposed. Furthermore, the second support portion may be contained along a part of its length within a space defined by the first support portion, the contained part of the second support portion rotatable within the space defined by the first support portion. The first support portion may comprise a sleeve which defines a substantially cylindrical space. The contained part of the second support portion may define an external surface of substantially the same circular cross-section along its length, the contained part of the second support portion received snugly within the substantially cylindrical space.

Further embodiments of the further aspect of the present invention may comprise one or more features of the first aspect of the present invention.

### Brief Description of Drawings

Further features and advantages of the present invention will become apparent from the following specific description, which is given by way of example only and with reference to the accompanying drawings, in which:
Figure 1 is a perspective view of an insertion instrument according to an embodiment of the present invention;
Figure 2 is an exploded perspective view of the insertion instrument of Figure 1;
Figure 3A is a perspective view of the insertion instrument of Figure 1 with rotation in a first direction about the longitudinal axis;
Figure 3B is a perspective view of the insertion instrument of Figure 1 with rotation in a second direction about the longitudinal axis;
Figure 4 contains a side view of the insertion instrument of Figure 1 at each of five steps in use of the insertion instrument;
Figure 5 contains a perspective view of the insertion instrument at each of the five steps shown in Figure 4;
Figure 6A is a perspective view of a superior or inferior plate;
Figure 6B is a view of the superior or inferior plate of Figure 6A from one side;
Figure 6C is a view of the superior or inferior plate of Figure 6A from an opposite side; and
Figure 6D is a plan view of the superior or inferior plate of Figure 6A.

### Description of Embodiments

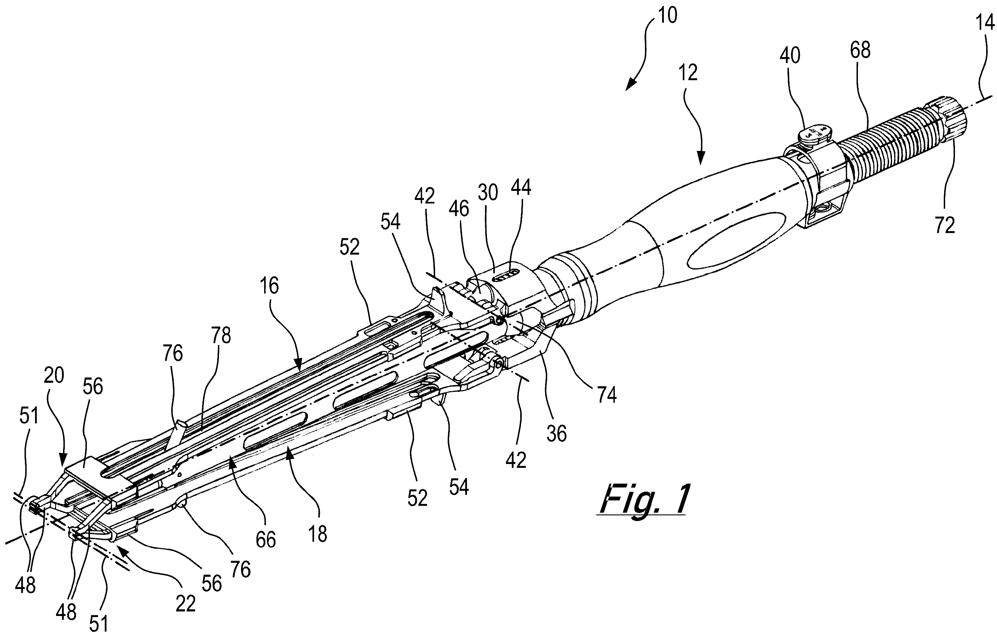

A perspective view of an insertion instrument 10 according to an embodiment of the present invention is shown in Figure 1. The insertion instrument 10 comprises a hand grippable support 12 which defines a longitudinal axis 14. The hand grippable support 12 defines a hand grippable surface which is contoured along its length, i.e. along the longitudinal axis 14, to thereby provide for improved grip by the surgeon. The insertion instrument 10 further comprises a first elongate arm 16 and a second elongate arm 18. The first elongate arm 16 is mounted at its proximal end on the hand grippable support 12 such that the arm extends from the hand grippable support along the longitudinal axis 14. As described further below, the first elongate arm 16 is configured at its distal end 20 to grip a superior plate of a modular OLIF device. The second elongate arm 18 is mounted at its proximal end on the hand grippable support 12 such that the arm extends from the hand grippable support along the longitudinal axis 14. As described further below, the second elongate arm is configured at its distal end 22 to grip an inferior plate of the modular OLIF device. As may be seen from Figure 1, the distal ends 20, 22 of the first and second elongate arms 16, 18 oppose each other whereby, in use, an inferior surface of the superior plate and a superior surface of the inferior plate face each other.

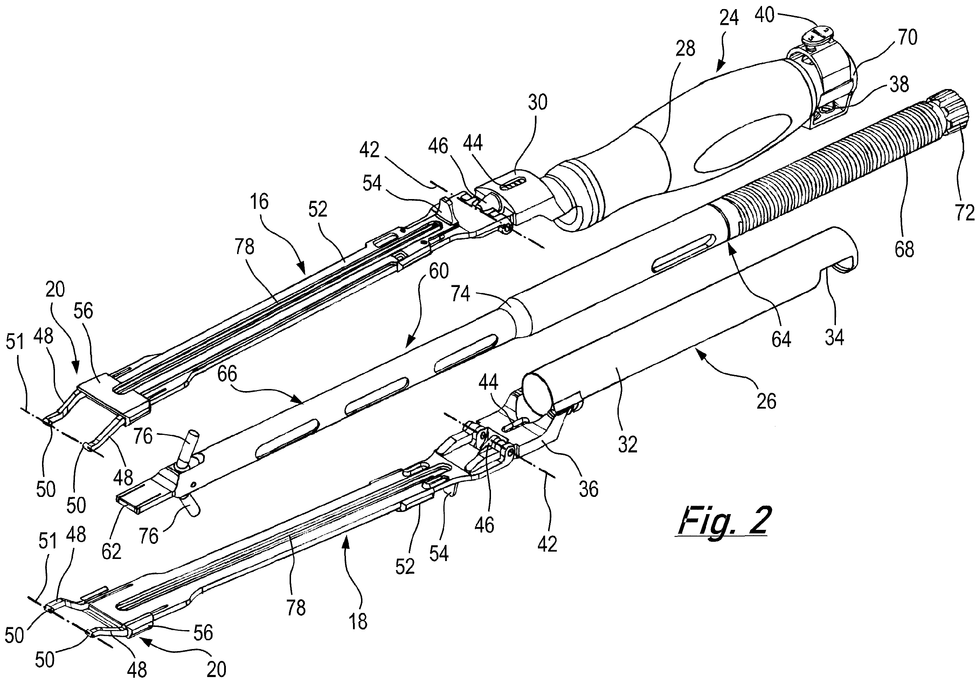

Referring now to the exploded view shown in Figure 2, the hand grippable support 12 comprises a first support portion 24 and a second support portion 26. The first support portion 24 comprises a sleeve 28 which defines a substantially cylindrical space. The exterior surface of the sleeve 28 defines the hand grippable surface described above. The first support portion 24 further comprises a first arm mounting bracket 30 which is immovably attached to and extends from an end of the sleeve 28. As described further below, the first arm 16 is attached to the first arm mounting bracket 30 by way of a hinge. The second support portion 26 comprises a tube 32 which defines an external surface of substantially the same circular cross-section along its length with the dimension of the cross-section sized such that the tube is a snug fit within the cylindrical space of the sleeve 28 while allowing for rotation of tube within the cylindrical space. The first and second support portions 24, 26 are thus coaxially disposed. The tube 32 has a cut out portion 34 towards its proximal end which engages with a profile (not shown) defined by the sleeve 28 to determine the extent of an arc of rotation of the tube 32 within the sleeve. The second support portion 26 further comprises a second arm mounting bracket 36 which is immovably attached to and extends from an end of the tube 32. As described further below, the second arm 18 is attached to the second arm mounting bracket 36 by way of a hinge. The first support portion 24 further comprises a spring arrangement 38 and a button 40 which is fixed attached on an arcuate plate which is mounted on the first support portion for movement towards and away from the longitudinal axis. The spring arrangement 38 applies a bias against the arcuate plate to urge the arcuate plate away from the longitudinal axis. Pressing on the button 40 moves the arcuate plate towards the longitudinal axis against the spring bias. Movement of the arcuate plate towards the longitudinal axis releases the first and second support portions 24, 26, and the core loader 60 (which is described below) from one another. When no pressure is applied to the button, a formation 70 defined by the arcuate plate threadedly engages with the threaded portion 68 on the core loader proximal portion 64, as described further below.

The hinge between each of the first and second mounting brackets 30, 36 and the respective one of the first and second arms 16, 18 will now be described. The arm 16, 18 is configured at its proximal end to form with the mounting bracket 30, 36 a hinged joint which provides for rotation of the arm around an arm rotation axis 42. The arm rotation axis 42 is substantially orthogonal to the longitudinal axis 14 and to a direction of separation of the first and second arms from each other. Each hinge allows for the respective arm 16, 18 to rotate relative to the respective mounting bracket 30, 36 whereby a separation between the distal ends of the arms may change by rotation of one, other or both of the arms. Each of the first and second mounting brackets 30, 36 comprises an arm spring 44 which exerts a spring bias along the longitudinal axis 14 towards the respective arm 16, 18. The spring bias is exerted on a biasing member 46 which bears against the respective arm 16, 18 such that the whole arm rotates about the arm rotation axis 42 and distal end of the arm is urged pivotally towards the distal end of the other arm. The two arm springs 44 and biasing members 46 are thus operative to urge the distal ends of the first and second arms 16, 18 pivotally towards each other. Increase in separation of the distal ends of the first and second arms 16, 18 is therefore against the bias exerted by the two arm springs 44 and biasing members 46. First and second sides of the first arm 16 are at opposite ends of the hinge of the first arm along the respective arm rotation axis 42. Third and fourth sides of the second arm 18 are at opposite ends of the hinge of the second arm along the respective arm rotation axis 42.

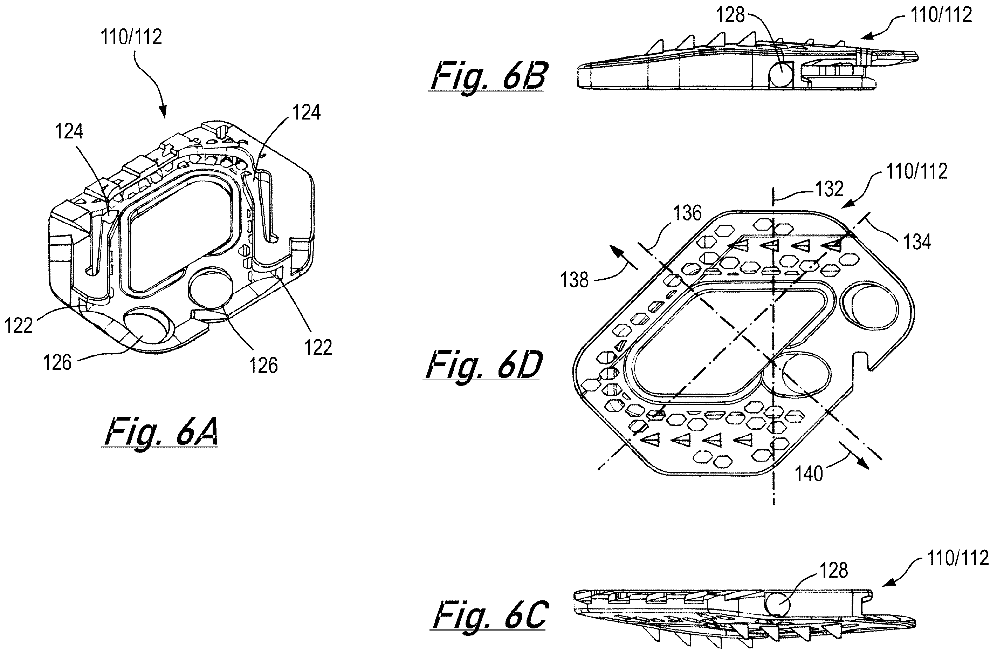

The distal end 20 of each of the first and second arms 16, 18 will now be described. The distal end 20 comprises two legs 48 which extend along the longitudinal axis 14. The two legs 48 are spaced apart in a direction orthogonal to the longitudinal axis 14 and to a direction of separation of the first and second arms 16, 18, i.e. the two legs are spaced apart in a direction parallel to the arm rotation axis 42. Each leg 48 has at its distal end a leg protrusion 50 which extends parallel to the arm rotation axis 42. The leg protrusions 50 of the two legs 48 are in registration with each other and extend towards each other. As described below with reference to Figures 6B and 6C, the superior and inferior plates define two recesses which are in registration with each other in opposite sides of the plate. Each leg protrusion 50 at a distal end 20 is shaped for rotatable reception in a respective one of the two recesses in a superior or inferior plate whereby the plate is gripped by the legs 48 and is rotatable relative to the gripped legs around a plate axis of rotation 51. The plate axis of rotation 51 is substantially parallel to the arm rotation axis 42.

Each of the first and second arms 16, 18 comprises an elongate leg separation member 52 which is mounted on the arm for movement thereon along the longitudinal axis 14. The leg separation member 52 defines a protrusion 54 at the proximal end of the leg separation member near the hand grippable support 12. The protrusion 54 is shaped and sized to engage with the surgeon's digit whereby the leg separation member 52 can be moved along the arm towards the hand grippable support 12 by the surgeon pressing on the protrusion. The leg separation member 52 has a separation sleeve 56 at a distal end of the leg separation member. The separation sleeve 56 is shaped and sized to slide over the distal end of the arm 16, 18 and to bear against a shoulder defined on each of the legs 48. When the separation sleeve 56 bears against the shoulders of the two legs 48 the two legs are bent towards each other against their inherent spring bias whereby the two legs engage with and grip a superior or inferior plate. Retraction of the separation sleeve 56 by the surgeon pressing against the protrusion 54 moves the separation sleeve towards the hand grippable support 12 and away from the shoulder defined on each of the legs 48. When the pressure has been released from the shoulders, the legs 48 return to their straightened condition under inherent spring bias whereby the legs spring apart and are disengaged from the gripped superior or inferior plate. A gripped superior or inferior plate is thus released from the insertion instrument 10 when the gripped superior or inferior plate has been installed in an intervertebral space.

The insertion instrument 10 further comprises an elongate core loader 60 which is configured at its distal end 62 to grip a core component of the OLIF device. In use, the core loader 60 inserts the core component between the superior and inferior plates after the superior and inferior plates have been inserted into the intervertebral space. The core loader 60 comprises a core loader proximal portion 64 which extends from a proximal end of the core loader along the core loader. The core loader proximal portion 64 is shaped and sized to be received in and move along the tube 32 of the second support portion 26. The core loader 60 is thus moved along the longitudinal axis 14 for insertion of the core component between the superior and inferior plates. The core loader 60 further comprises a core loader distal portion 66 which extends from an end of the core loader proximal portion 64 to the distal end 62 of the core loader. The core loader distal portion 66 extends along the longitudinal axis 14 between the first and second arms 16, 18.

The core loader proximal portion 64 define a threaded portion 68 which threadedly engages with a formation 70 defined by the arcuate plate fixedly attached to the button 40. Rotation of the core loader proximal portion 64 relative to the second support portion 24 by the surgeon rotating a knob at the proximal end of the core loader moves the core loader along the longitudinal axis. The core loader proximal portion 64 and the core loader distal portion 66 are attached to each other by a rotatable coupling 74 whereby the core loader distal portion 66 is decoupled from rotation of the core loader proximal portion. The core loader distal portion 66 has two protrusions 76 which extend in opposite directions in a direction of separation of the first and second arms 16, 18. Each of the first and second arms 16, 18 defines a slot 78 which extends along the longitudinal axis 14. One of the two protrusions 76 is received in the slot defined by the first arm 16 and the other of the protrusions 76 is received in the slot defined by the second arm 18. When the surgeon rotates the core loader proximal portion 64 relative to the second support portion 24, the rotating core loader proximal portion 64 moves the core loader distal portion 66 along the longitudinal axis 14. As the core loader distal portion 66 moves along the longitudinal axis 14, the two protrusions 76 travel along their respective slot 78 and abut against a side of the respective slot to thereby prevent the core loader distal portion rotating with the core loader proximal portion 64.

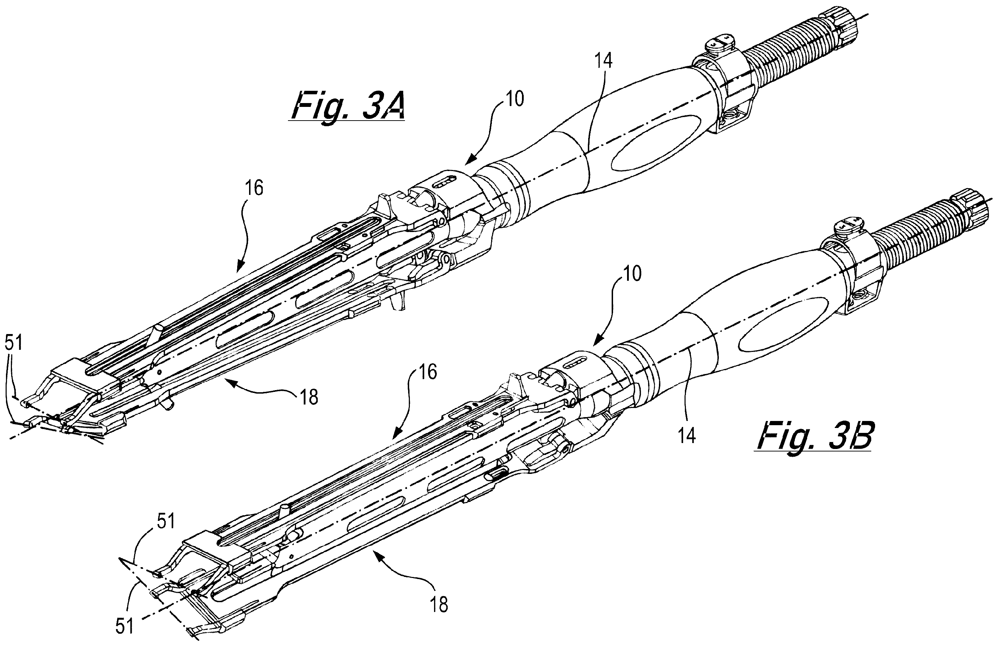

Figures 3A and 3B provide perspective views of the insertion instrument 10 of Figures 1 and 2 when the first and second arms 16, 18 are rotated relative to each other around the longitudinal axis 14. Relative rotation of the first and second arms 16, 18 allows the insertion instrument 10 to adapt to a core component which is shaped as a frustrum of a wedge and when the core component is inserted by the core loader between superior and inferior plates gripped by the insertion instrument. Figure 3A shows the insertion instrument 10 after rotation in a first direction and Figure 3B shows the insertion instrument after rotation in a second direction opposite the first direction. Rotation in the first or second direction causes gripped superior and inferior plates to be inclined to each other, as is evident from the inclination of the two plate axes of rotation 51 to each other in Figures 3A and 3B.

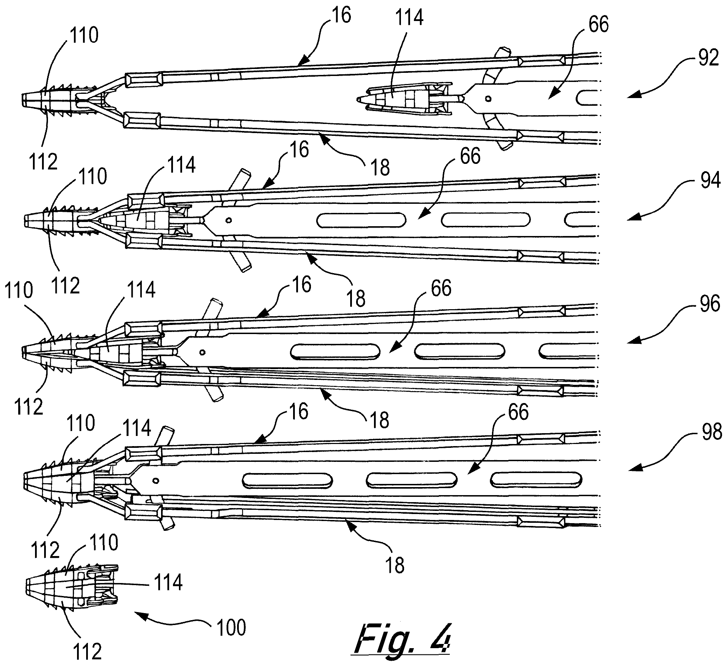

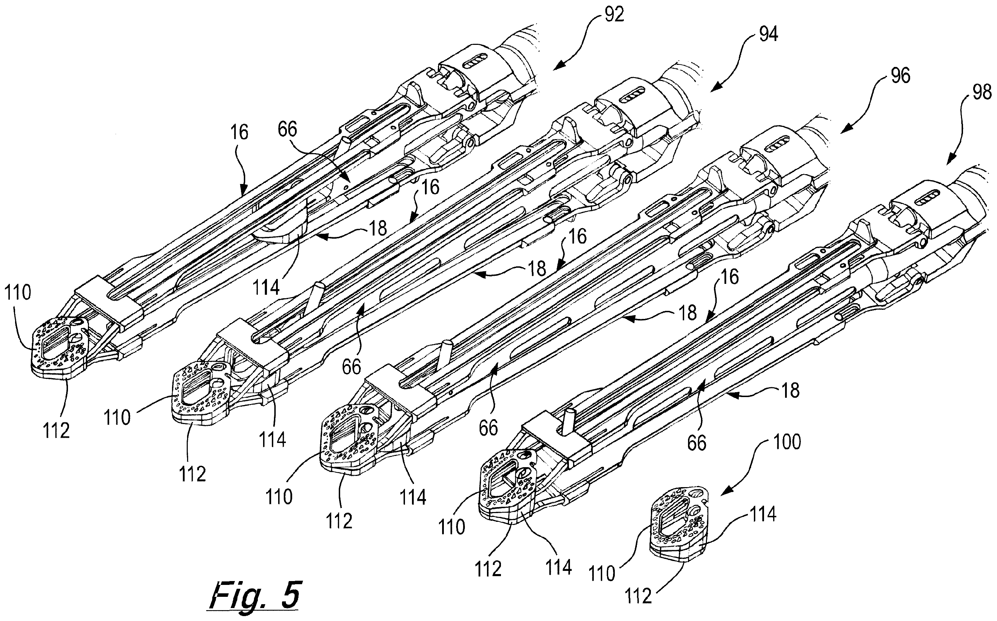

Figures 4 and 5 show a side view and a perspective view respectively of the insertion instrument 10 of Figures 1 at each of five steps in use of the insertion instrument. **In** each of the first four steps 92, 94, 96, 98 the superior plate 110 is gripped by the first arm 16 and the inferior plate 112 is gripped by the second arm 18. Further to this, in each of the first four steps 92, 94, 96, 98 the core component 114 is supported on the distal end of the core loader 66. At the first step 92 the insertion instrument has been used to locate the gripped superior and inferior plates 110, 112 in the intervertebral space. At the second step 94 the surgeon has advanced the core component 114 by way of the core loader 66 to between the distal ends of the first and second arms 16, 18. Upon further advancing of the core component 114, the core component starts to push the distal ends and gripped superior and inferior plates 110, 112 apart 96. As the core component 114 is inserted between and engages with the gripped superior and inferior plates 110, 112 the wedge shape of the core component forces the superior and inferior plates 110, 112 to rotate relative to each other about the longitudinal axis 14 whereby the first and second arms adopt one of the relatively rotated dispositions shown in Figures 3A and 3B. When the core component 114 has been fully inserted between the superior and inferior plates 110, as indicated by reference numeral 98, the surgeon operates the two separation members 52 to disengage the superior and inferior plates 110 from their respective arms 16, 18 to leave the assembled OLIF superior and inferior plates 110 and the core component 114 in the intervertebral space, as indicated by reference numeral 100.

Four different views of a superior or inferior plate 110, 112 are shown in Figures 6A to 6D. Figure 6A is a perspective view of the plate 110, 112. The plate 110, 112 defines a channel 122 at each side for slidable inter-engagement with the core component 114. The plate 110, 112 also defines two barb ended sprung arms 124 adjacent a respective channel 122 which are operative to lock the core component and plate together when the core component is fully inserted. The plate 110, 112 further defines two apertures 126 each for receiving a respective bone screw. Figures 6B and 6C show left and right hand views respectively of the plate 110, 112. Each of the left and right hand sides of the plate 110, 112 defines a cylindrical recess 128 with the two recesses 128 in registration with each other. The two recesses 128 receive a respective one of the leg protrusions 50 at the distal end of an arm 16, 18 whereby the plate 110, 112 is gripped by the arm.

Figure 6D is a plan view of the superior or inferior plate 110, 112. A first line 132 is the axis of rotation of the plate about the recesses 128, i.e. the plate axis of rotation 51. The insertion instrument 10 is introduced into the patient's body in a direction orthogonal to the axis of rotation of the plate about the recesses 132. A second line 134 is the medial-lateral direction of the patient and a third line 136 is the posterior-anterior direction of the patient. A first arrow 138 points in the posterior direction of the patient and a second arrow 140 points in the anterior direction of the patient. As may be appreciated from consideration of Figure 6D the direction of introduction of the insertion instrument 10 is at an oblique angle to the medial-lateral and posterior-anterior directions. Further to this, a wedge shaped core component has upper and lower surfaces which are inclined to each other in the posterior-anterior direction. Were the present insertion instrument unable to allow for rotation of the first and second arms 16, 18 about the longitudinal axis 14, the superior and inferior plates 110, 112 would be presented and held with their relative orientation different to the relative orientation of the upper and lower surfaces of the core component 114. This would make it difficult if not impossible for the core component 114 to be brought into inter-engagement with the superior and inferior plates 110, 112. Rotation of the first and second arms 16, 18 about the longitudinal axis 14 allows for the relative orientation of the superior and inferior plates 110, 112 to adopt to the relative orientation of the upper and lower surfaces of the core component 114 as the core component is inserted between the superior and inferior plates.

## Claims

1. An insertion instrument (10) for inserting a modular Oblique Lumbar Interbody Fusion (OLIF) device (100) into an intervertebral space, the insertion instrument comprising:
a hand grippable support (12) defining a longitudinal axis (14);
a first elongate arm (16) mounted at its proximal end on the hand grippable support and extending from the hand grippable support along the longitudinal axis, the first elongate arm configured at its distal end (20) to grip a superior plate (110) of the modular OLIF device;
a second elongate arm (18) mounted at its proximal end on the hand grippable support and extending from the hand grippable support along the longitudinal axis, the second elongate arm configured at its distal end (22) to grip an inferior plate (112) of the modular OLIF device, the distal ends of the first and second elongate arms opposing each other whereby, in use, an inferior surface of the superior plate and a superior surface of the inferior plate face each other, wherein
the proximal ends of the first and second arms are mounted on the hand grippable support for relative rotation of the first and second arms (16, 18) about the longitudinal axis (14), and
the hand grippable support (12) comprises a first support portion (24) on which the first arm (16) is mounted, and a second support portion (26) on which the second arm (18) is mounted, the first and second support portions coupled for their relative rotation about the longitudinal axis (14), relative rotation of the first and second support portions causing relative rotation of the first and second arms about the longitudinal axis,
**characterised in that** the first and second support portions (26, 26) are coaxially disposed.

2. The insertion instrument according to claim 1, wherein at least one of the first and second arms (16, 18) is configured at a location along its length for rotation of the distal end of the arm about an arm rotation axis (42) which is orthogonal to the longitudinal axis (14) and to a direction of separation of the first and second arms, rotation about the arm rotation axis changing separation between the distal ends (20, 22) of the first and second arms.

3. The insertion instrument according to claim 2, wherein the arm rotation axis (42) is at or adjacent the proximal end of the arm whereby the whole arm rotates about the arm rotation axis and the insertion instrument comprises a hinge which provides for rotation about the arm rotation axis, the hinge comprising first and second hinge parts which are hingedly coupled to each other, the first hinge part comprised in the proximal end of the arm and the second hinge part comprised in the hand grippable support.

4. The insertion instrument according to any one of the preceding claims, wherein the second support portion (26) is contained along a part of its length within a space defined by the first support portion (24), the contained part of the second support portion rotatable within the space defined by the first support portion.

5. The insertion instrument according to claim 4, wherein the first support portion (24) comprises a sleeve (28) which defines a substantially cylindrical space, the contained part of the second support portion (26) defining along its length an external surface of substantially the same circular cross-section as the substantially cylindrical space, the contained part of the second support portion received snugly within the substantially cylindrical space whereby the contained part is rotatable in the sleeve.

6. The insertion instrument according to claim 5, wherein an exterior surface of the sleeve (28) defines a hand grippable surface which is contoured along its length.

7. The insertion instrument according to claim 5 or 6, wherein the first support portion (24) further comprises a first arm mounting bracket (30), the first arm mounting bracket immovably attached to and extending from an end of the sleeve (28), the first arm (16) attached to the first arm mounting bracket.

8. The insertion instrument according to any one of claims 5 to 7, wherein the second support portion (26) further comprises a second arm mounting bracket (36), the second arm mounting bracket immovably attached to and extending from an end of the contained part of the second support portion, the second arm (18) attached to the second arm mounting bracket.

9. The insertion instrument according to any one of claims 5 to 8, wherein the second support portion (26) comprises a tube (32) which is received in the sleeve (28) of the first support portion (24), the tube constituting the contained part of the second support portion.

10. The insertion instrument according to claim 9 further comprising an elongate core loader (60) which is configured at its distal end to grip a core component (114) of the OLIF device, the core loader comprising a core loader proximal portion (64) which extends along the core loader from a proximal end of the core loader, the core loader proximal portion shaped and sized to be received in and moved along the tube (32) of the second support portion whereby the core loader is moved along the longitudinal axis (14).

11. The insertion instrument according to claim 10, wherein the core loader (60) further comprises a core loader distal portion (66) which extends from an end of the core loader proximal portion (64), the core loader distal portion received between the first and second arms (16, 18) when the core loader proximal portion is received in the tube (32) of the second support portion.

12. The insertion instrument according to claim 11, wherein the core loader proximal portion (64) defines a threaded portion (68) which threadedly engages with the second support portion (26), rotation of the core loader proximal portion (64) relative to the second support portion moving the core loader (60) along the longitudinal axis (14), the core loader further comprising a rotatable coupling (74) between the core loader proximal portion and the core loader distal portion.

13. The insertion instrument according to claim 12, wherein the core loader distal portion (66) comprises a rotation resisting formation (76) which bears against at least one of the first and second arms to thereby present resistance to rotation of the core loader distal portion with rotation of the core loader proximal portion (64) and whereby there is rotation about the rotatable coupling (74) of the core loader proximal portion relative to the core loader distal portion.

14. The insertion instrument according to claim 13, wherein the arm defines a slot (78) which extends in the longitudinal axis (14) and in which the rotation resisting formation (76) is received, the rotation resisting formation bearing against a side of the slot to present resistance to rotation of the core loader distal portion (66), and the rotation resisting formation moving along the slot as the core loader (60) moves along the longitudinal axis.

15. A kit for an OLIF procedure comprising an insertion instrument (10) according to any one of the preceding claims, a superior plate (110), an inferior plate (112), and at least one core component (114), the OLIF device constituted by the superior plate, the inferior plate, and the core component.

## Patentansprüche

1. Ein Einschubinstrument (10) zum Einschieben einer modularen schrägen lumbalen interkorporellen Fusionsvorrichtung (OLIF-Vorrichtung) (100) in einen Zwischenwirbelraum, wobei das Einschubinstrument Folgendes beinhaltet:
ein mit der Hand ergreifbares Stützelement (12), das eine Längsachse (14) definiert;
einen ersten länglichen Arm (16), der an seinem proximalen Ende an dem mit der Hand ergreifbaren Stützelement angebracht ist und sich von dem mit der Hand ergreifbaren Stützelement entlang der Längsachse erstreckt, wobei der erste längliche Arm an seinem distalen Ende (20) dazu ausgelegt ist, eine obere Platte (110) der modularen OLIF-Vorrichtung zu ergreifen;
einen zweiten länglichen Arm (18), der an seinem proximalen Ende an dem mit der Hand ergreifbaren Stützelement angebracht ist und sich von dem mit der Hand ergreifbaren Stützelement entlang der Längsachse erstreckt, wobei der zweite längliche Arm an seinem distalen Ende (22) dazu ausgelegt ist, eine untere Platte (112) der modularen OLIF-Vorrichtung zu ergreifen, wobei die distalen Enden des ersten und zweiten länglichen Arms einander gegenüberliegen, wodurch im Gebrauch eine untere Oberfläche der oberen Platte und eine obere Oberfläche der unteren Platte zueinander zeigen, wobei
die proximalen Enden des ersten und zweiten Arms zur relativen Rotation des ersten und zweiten Arms (16, 18) um die Längsachse (14) an dem mit der Hand ergreifbaren Stützelement angebracht sind, und
das mit der Hand ergreifbare Stützelement (12) einen ersten Stützelementanteil (24), an dem der erste Arm (16) angebracht ist, und einen zweiten Stützelementanteil (26), an dem der zweite Arm (18) angebracht ist, beinhaltet, wobei der erste und zweite Stützelementanteil für ihre relative Rotation um die Längsachse (14) gekoppelt sind, wobei eine relative Rotation des ersten und zweiten Stützelementanteils eine relative Rotation des ersten und zweiten Arms um die Längsachse bewirkt,
**dadurch gekennzeichnet, dass** der erste und zweite Stützelementanteil (26, 26) koaxial angeordnet sind.

2. Einschubinstrument gemäß Anspruch 1, wobei mindestens einer von dem ersten und zweiten Arm (16, 18) dazu ausgelegt ist, an einem Ort entlang seiner Länge zur Rotation des distalen Endes des Arms um eine Armrotationsachse (42) ausgelegt ist, die senkrecht zu der Längsachse (14) und zu einer Richtung der Trennung des ersten und zweiten Arms ist, wobei die Rotation um die Armrotationsachse die Trennung zwischen den distalen Enden (20, 22) des ersten und zweiten Arms verändert.

3. Einschubinstrument gemäß Anspruch 2, wobei die Armrotationsachse (42) an dem oder neben dem proximalen Ende des Arms ist, wodurch sich der ganze Arm um die Armrotationsachse dreht, und wobei das Einschubinstrument ein Scharnier beinhaltet, das eine Rotation um die Armrotationsachse bereitstellt, wobei das Scharnier erste und zweite Scharnierteile beinhaltet, die schwenkbar miteinander gekoppelt sind, wobei das erste Scharnierteil in dem proximalen Ende des Arms enthalten ist und das zweite Scharnierteil in dem mit der Hand ergreifbaren Stützelement enthalten ist.

4. Einschubinstrument gemäß einem der vorhergehenden Ansprüche, wobei der zweite Stützelementanteil (26) entlang eines Teils seiner Länge innerhalb eines Raums enthalten ist, der durch den ersten Stützelementanteil (24) definiert wird, wobei der enthaltene Teil des zweiten Stützelementanteils innerhalb des durch den ersten Stützelementanteil definierten Raums rotierbar ist.

5. Einschubinstrument gemäß Anspruch 4, wobei der erste Stützelementanteil (24) eine Hülse (28) beinhaltet, die einen im Wesentlichen zylinderförmigen Raum definiert, wobei der enthaltene Teil des zweiten Stützelementanteils (26) entlang seiner Länge eine äußere Oberfläche des im Wesentlichen gleichen kreisförmigen Querschnitts definiert wie der im Wesentlichen zylinderförmige Raum, wobei der enthaltene Teil des zweiten Stützelementanteils passgenau innerhalb des im Wesentlichen zylinderförmigen Raums aufgenommen wird, wodurch der enthaltene Teil in der Hülse rotierbar ist.

6. Einschubinstrument gemäß Anspruch 5, wobei eine äußere Oberfläche der Hülse (28) eine mit der Hand ergreifbare Oberfläche definiert, die entlang ihrer Länge profiliert ist.

7. Einschubinstrument gemäß Anspruch 5 oder 6, wobei der erste Stützelementanteil (24) ferner eine erste Armbefestigungshalterung (30) beinhaltet, wobei die erste Armbefestigungshalterung unbeweglich an einem Ende der Hülse (28) befestigt ist und sich davon erstreckt, wobei der erste Arm (16) an der ersten Armbefestigungshalterung befestigt wird.

8. Einschubinstrument gemäß einem der Ansprüche 5 bis 7, wobei der zweite Stützelementanteil (26) ferner eine zweite Armbefestigungshalterung (36) beinhaltet, wobei die zweite Armbefestigungshalterung unbeweglich an einem Ende des enthaltenen Teils des zweiten Stützelementanteils befestigt ist und sich davon erstreckt, wobei der zweite Arm (18) an der zweiten Armbefestigungshalterung befestigt wird.

9. Einschubinstrument gemäß einem der Ansprüche 5 bis 8, wobei der zweite Stützelementanteil (26) ein Rohr (32) beinhaltet, das in der Hülse (28) des ersten Stützelementanteils (24) aufgenommen wird, wobei das Rohr den enthaltenen Teil des zweiten Stützelementanteils bildet.

10. Einschubinstrument gemäß Anspruch 9, das ferner einen länglichen Kernlader (60) beinhaltet, der an seinem distalen Ende dazu ausgelegt ist, eine Kernkomponente (114) der OLIF-Vorrichtung zu ergreifen, wobei der Kernlader einen proximalen Kernladeranteil (64) beinhaltet, der sich entlang des Kernladers von einem proximalen Ende des Kernladers erstreckt, wobei der proximale Kernladeranteil zum Aufnehmen in und Bewegen entlang des Rohrs (32) des zweiten Stützelementanteils gestaltet und dimensioniert ist, wodurch der Kernlader entlang der Längsachse (14) bewegt wird.

11. Einschubinstrument gemäß Anspruch 10, wobei der Kernlader (60) ferner einen distalen Kernladeranteil (66) beinhaltet, der sich von einem Ende des proximalen Kernladeranteils (64) erstreckt, wobei der distale Kernladeranteil zwischen dem ersten und zweiten Arm (16, 18) aufgenommen wird, wenn der proximale Kernladeranteil in dem Rohr (32) des zweiten Stützelementanteils aufgenommen wird.

12. Einschubinstrument gemäß Anspruch 11, wobei der proximale Kernladeranteil (64) einen gewindeten Anteil (68) definiert, der gewindeten Eingriff mit dem zweiten Stützelementanteil (26) nimmt, wobei eine Rotation des proximalen Kernladeranteils (64) relativ zu dem zweiten Stützelementanteil den Kernlader (60) entlang der Längsachse (14) bewegt, wobei der Kernlader ferner eine rotierbare Kopplung (74) zwischen dem proximalen Kernladeranteil und dem distalen Kernladeranteil beinhaltet.

13. Einschubinstrument gemäß Anspruch 12, wobei der distale Kernladeranteil (66) eine rotationsfeste Ausformung (76) beinhaltet, die gegen mindestens einen von dem ersten und zweiten Arm drückt, um somit Widerstand gegen eine Rotation des distalen Kernladeranteils bei Rotation des proximalen Kernladeranteils (64) zu bieten, und wodurch es eine Rotation um die rotierbare Kopplung (74) des proximalen Kernladeranteils relativ zu dem distalen Kernladeranteil gibt.

14. Einschubinstrument gemäß Anspruch 13, wobei der Arm einen Schlitz (78) definiert, der sich in der Längsachse (14) erstreckt und in dem die rotationsfeste Ausformung (76) aufgenommen wird, wobei die rotationsfeste Ausformung gegen eine Seite des Schlitzes drückt, um Widerstand gegen eine Rotation des distalen Kernladeranteils (66) zu bieten, und wobei sich die rotationsfeste Ausformung entlang des Schlitzes bewegt während sich der Kernlader (60) entlang der Längsachse bewegt.

15. Ein Kit für einen OLIF-Eingriff, das ein Einschubinstrument (10) gemäß einem der vorhergehenden Ansprüche, eine obere Platte (110), eine untere Platte (112) und mindestens eine Kernkomponente (114) beinhaltet, wobei die OLIF-Vorrichtung durch die obere Platte, die untere Platte und die Kernkomponente gebildet wird.

## Revendications

1. Instrument d'insertion (10) pour insérer un dispositif de spondylodèse lombaire par voie oblique (OLIF) modulaire (100) dans un espace intersomatique, l'instrument d'insertion comprenant :
un support préhensible (12) définissant un axe longitudinal (14) ;
un premier bras allongé (16) fixé, au niveau de son extrémité proximale, sur le support préhensible et s'étendant à partir du support préhensible le long de l'axe longitudinal, le premier bras allongé étant conçu, au niveau de son extrémité distale (20), pour saisir une plaque supérieure (110) du dispositif OLIF modulaire ;
un second bras allongé (18) fixé, au niveau de son extrémité proximale, sur le support préhensible et s'étendant à partir du support préhensible le long de l'axe longitudinal, le second bras allongé étant conçu, au niveau de son extrémité distale (22), pour saisir une plaque inférieure (112) du dispositif OLIF modulaire, les extrémités distales des premier et second bras allongés se faisant face de telle sorte que, durant l'utilisation, une surface inférieure de la plaque supérieure et une surface supérieure de la plaque inférieure soient disposées en regard l'une de l'autre,
les extrémités proximales des premier et second bras étant fixées sur le support préhensible de façon à permettre une rotation relative des premier et second bras (16, 18) autour de l'axe longitudinal (14), et
le support préhensible (12) comprenant une première partie de support (24) sur laquelle est fixé le premier bras (16), et une seconde partie de support (26) sur laquelle est fixé le second bras (18), les première et seconde parties de support étant accouplées de façon à permettre leur rotation relative autour de l'axe longitudinal (14), cette rotation relative des première et seconde parties de support provoquant une rotation relative des premier et second bras autour de l'axe longitudinal,
**caractérisé en ce que** les première et seconde parties de support (26, 26) sont disposées de manière coaxiale.

2. Instrument d'insertion selon la revendication 1, dans lequel au moins un des premier et second bras (16, 18) est conçu, à un emplacement le long de sa longueur, de façon à permettre une rotation de l'extrémité distale du bras autour d'un axe de rotation de bras (42) qui est orthogonal à l'axe longitudinal (14) et à une direction de séparation des premier et second bras, cette rotation autour de l'axe de rotation de bras changeant la séparation entre les extrémités distales (20, 22) des premier et second bras.

3. Instrument d'insertion selon la revendication 2, dans lequel l'axe de rotation de bras (42) se trouve au niveau ou près de l'extrémité proximale du bras, de telle sorte que la totalité du bras tourne autour de l'axe de rotation de bras et l'instrument d'insertion comprenant une charnière qui permet la rotation autour de l'axe de rotation de bras, la charnière comprenant des première et seconde parties de charnière accouplées en charnière, la première partie de charnière faisant partie de l'extrémité proximale du bras et la seconde partie de charnière faisant partie du support préhensible.

4. Instrument d'insertion selon l'une quelconque des revendications précédentes, dans lequel la seconde partie de support (26) est logée, le long d'une section de sa longueur, à l'intérieur d'un espace défini par la première partie de support (24), la section logée de la seconde partie de support pouvant tourner à l'intérieur de l'espace défini par la première partie de support.

5. Instrument d'insertion selon la revendication 4, dans lequel la première partie de support (24) comprend un manchon (28) qui définit un espace sensiblement cylindrique, la section logée de la seconde partie de support (26) définissant, le long de sa longueur, une surface externe présentant sensiblement la même section transversale circulaire que l'espace sensiblement cylindrique, la section logée de la seconde partie de support étant reçue avec ajustement serré à l'intérieur de l'espace sensiblement cylindrique de telle sorte que la section logée puisse tourner dans le manchon.

6. Instrument d'insertion selon la revendication 5, dans lequel une surface extérieure du manchon (28) définit une surface préhensible qui est profilée le long de sa longueur.

7. Instrument d'insertion selon la revendication 5 ou 6, dans lequel la première partie de support (24) comprend, en outre, une pièce de fixation de premier bras (30), la pièce de fixation de premier bras étant attachée fixement à une extrémité du manchon (28) et s'étendant à partir de celle-ci, le premier bras (16) étant attaché à la pièce de fixation de premier bras.

8. Instrument d'insertion selon l'une quelconque des revendications 5 à 7, dans lequel la seconde partie de support (26) comprend, en outre, une pièce de fixation de second bras (36), la pièce de fixation de second bras étant attachée fixement à une extrémité de la section logée de la seconde partie de support et s'étendant à partir de celle-ci, le second bras (18) étant attaché à la pièce de fixation de second bras.

9. Instrument d'insertion selon l'une quelconque des revendications 5 à 8, dans lequel la seconde partie de support (26) comprend un tube (32) qui est reçu dans le manchon (28) de la première partie de support (24), le tube constituant la section logée de la seconde partie de support.

10. Instrument d'insertion selon la revendication 9, comprenant, en outre, un chargeur de composant central allongé (60) qui est conçu, au niveau de son extrémité distale, pour saisir un composant central (114) du dispositif OLIF, le chargeur de composant central comprenant une partie proximale de chargeur de composant central (64) qui s'étend le long du chargeur de composant central à partir d'une extrémité proximale du chargeur de composant central, la partie proximale de chargeur de composant central présentant une forme et des dimensions lui permettant d'être reçue dans le tube (32) de la seconde partie de support et déplacée le long de celui-ci de telle sorte que le chargeur de composant central soit déplacé le long de l'axe longitudinal (14).

11. Instrument d'insertion selon la revendication 10, dans lequel le chargeur de composant central (60) comprend, en outre, une partie distale de chargeur de composant central (66) qui s'étend à partir d'une extrémité de la partie proximale de chargeur de composant central (64), la partie distale de chargeur de composant central étant reçue entre les premier et second bras (16, 18) lorsque la partie proximale de chargeur de composant central est reçue dans le tube (32) de la seconde partie de support.

12. Instrument d'insertion selon la revendication 11, dans lequel la partie proximale de chargeur de composant central (64) définit une partie filetée (68) qui entre en prise par vissage avec la seconde partie de support (26), une rotation de la partie proximale de chargeur de composant central (64) relativement à la seconde partie de support déplaçant le chargeur de composant central (60) le long de l'axe longitudinal (14), le chargeur de composant central comprenant, en outre, un accouplement rotatif (74) entre la partie proximale de chargeur de composant central et la partie distale de chargeur de composant central.

13. Instrument d'insertion selon la revendication 12, dans lequel la partie distale de chargeur de composant central (66) comprend une structure antirotation (76) qui vient en appui contre au moins l'un des premier et second bras de façon à exercer une résistance à l'encontre d'une rotation de la partie distale de chargeur de composant central lors d'une rotation de la partie proximale de chargeur de composant central (64) et de telle sorte qu'une rotation de la partie proximale de chargeur de composant central relativement à la partie distale de chargeur de composant central se produise autour de l'accouplement rotatif (74).

14. Instrument d'insertion selon la revendication 13, dans lequel le bras définit une fente (78) qui s'étend le long de l'axe longitudinal (14) et dans laquelle est reçue la structure antirotation (76), la structure antirotation venant en appui contre un côté de la fente afin d'exercer une résistance à l'encontre d'une rotation de la partie distale de chargeur de composant central (66), et la structure antirotation se déplaçant le long de la fente à mesure que le chargeur de composant central (60) se déplace le long de l'axe longitudinal.

15. Trousse pour une intervention OLIF comprenant un instrument d'insertion (10) selon l'une quelconque des revendications précédentes, une plaque supérieure (110), une plaque inférieure (112) et au moins un composant central (114), le dispositif OLIF étant constitué par la plaque supérieure, la plaque inférieure et le composant central.
